# EUROPÄISCHE PATENTSCHRIFT

(11) **EP 3 256 187 B1**
(45) Veröffentlichungstag und Bekanntmachung des Hinweises auf die Patenterteilung: **26.05.2021**
(21) Anmeldenummer: 16705467.5
(22) Anmeldetag: 12.02.2016
(51) Int. Cl.: A61M 1/36

(54) **VERFAHREN ZUM ÜBERPRÜFEN EINES VERBINDUNGSZUSTANDES EINER BLUTBEHANDLUNGSVORRICHTUNG MIT EINEM BLUTSCHLAUCHSATZ, SOWIE VORRICHTUNGEN**
METHOD FOR TESTING A CONNECTION STATE OF A BLOOD TREATMENT APPARATUS COMPRISING A BLOOD TUBE SET, AND APPARATUSES
PROCÉDÉ DE CONTRÔLE DE L'ÉTAT D'UNE LIAISON ENTRE UN DISPOSITIF DE TRAITEMENT DU SANG ET UN SET DE LIGNES À SANG, ET DISPOSITIFS

(30) Priorität: 12.02.2015 DE 102015102040
(43) Veröffentlichungstag der Anmeldung: 20.12.2017
(73) Patentinhaber: Fresenius Medical Care Deutschland GmbH, 61352 Bad Homburg (DE)
(72) Erfinder: BEDEN, Josef, 55252 Mainz-Kastel (DE)
(74) Vertreter: Bobbert & Partner Patentanwälte PartmbB
(86) Internationale Anmeldenummer: PCT/EP2016/053032
(87) Internationale Veröffentlichungsnummer: WO 2016/128555

(56) Entgegenhaltungen:
- WO-A1-2014/099779
- US-A1- 2009 152 179
- US-A1- 2012 083 726

## Beschreibung

Die vorliegende Erfindung betrifft ein Verfahren gemäß Anspruch 1. Sie betrifft zudem eine medizinische Blutbehandlungsvorrichtung gemäß Anspruch 8..

Aus der WO 2014/099779 A1 ist die Erkennung der Verbindung von Blutsatzkomponenten bekannt.

In der US 2012/083726 A1 sind ein Verfahren und eine Vorrichtung zum Überwachen eines Fluidsystems einer extrakorporalen Blutbehandlungsvorrichtung offenbart.

In der US 2009/152179 A1 sind ein Verfahren zum Freiblasen eines benetzen Hydrophobfilters sowie eine Vorrichtung zum Durchführen dieses Verfahrens offenbart.

Aus der WO 2013/017236 A1 sind maschinelle Tests zum Überprüfen von Funktion und Zuverlässigkeit von medizinischen Funktionseinrichtungen wie extrakorporalen Blutschläuchen, Blutkassetten und dergleichen oder deren korrekte Verbindung mit der Blutbehandlungsvorrichtung bekannt. Ein solcher Test kann beispielsweise ein Druckhaltetest sein. Er wird durchgeführt, nachdem die in Frage stehende Funktionseinrichtung mit der Blutbehandlungsvorrichtung verbunden ist, also nachdem die Blutbehandlungsvorrichtung bereits mit der Funktionseinrichtung aufgerüstet ist, und bevor die Behandlung des Patienten beginnt.

Eine Aufgabe der vorliegenden Erfindung ist es, ein weiteres Verfahren für eine solche Überprüfung einer Verbindung eines Blutschlauchsatzes an der Blutbehandlungsvorrichtung vorzuschlagen. Zudem soll eine Blutbehandlungsvorrichtung angegeben werden.

Die erfindungsgemäße Aufgabe wird durch ein Verfahren mit den Merkmalen des Anspruchs 1 gelöst. Sie wird ferner gelöst mittels der Blutbehandlungsvorrichtung mit den Merkmalen des Anspruchs 8.

Erfindungsgemäß wird somit ein Verfahren vorgeschlagen zum Überprüfen einer Verbindung zwischen einem Druckluftausgang einer Blutbehandlungsvorrichtung und einer Druckmessleitung eines extrakorporalen Blutschlauchsatzes.

Das Verfahren umfasst das Bereitstellen einer Blutbehandlungsvorrichtung. Diese weist eine Druckluftleitung und eine mit dieser in Fluidkommunikation stehende Drucklufteinrichtung auf, wobei letztere zum Erzeugen von Druck und einer Luftströmung innerhalb der Druckluftleitung dient.

Die Blutbehandlungsvorrichtung weist einen sowohl mit der Druckluftleitung als auch mit einem Äußeren der Blutbehandlungsvorrichtung in Fluidkommunikation stehenden Druckluftausgang auf.

Der Druckluftausgang ist ausgestaltet, um mit einer Druckmessleitung verbindbar zu sein. Hier ist er beispielsweise als Port, Verbinder, Luer-Konnektor (oder als männlicher oder weiblicher Part eines Luer-Konnektors) oder dergleichen ausgestaltet oder weist ein derartiges Verbindungselement auf.

Die Blutbehandlungsvorrichtung weist ferner einen Drucksensor auf, welcher angeordnet ist zum Messen des am Druckluftausgang oder in der Druckluftleitung herrschenden Drucks.

Das erfindungsgemäße Verfahren umfasst weiter ein Aufbauen eines Drucks, vorzugsweise eines Luftdrucks, und einer Luftströmung in der Druckluftleitung und/oder am Druckluftausgang. Der Druck und die Luftströmung werden mittels der Drucklufteinrichtung erzeugt.

Das erfindungsgemäße Verfahren umfasst ferner ein Messen eines am Druckluftausgang oder in der Druckluftleitung herrschenden Drucks, beispielsweise als Absolutwert oder als Druckveränderung über der Zeit. Gemessen wird dies jeweils mittels des Drucksensors.

Schließlich umfasst das erfindungsgemäße Verfahren ein Auswerten eines Anstiegs des gemessenen Drucks, beispielsweise gegenüber einem Ausgangswert, oder eines Anstiegs des Drucks über der Zeit. Das Auswerten erfolgt vorzugsweise jeweils mittels eines Vergleichs des gemessenen Drucks oder eines gemessenen Druckanstiegs mit zuvor gemessenen oder gespeicherten Werten, Schwellenwerten, Bereichen oder Verläufen.

Die von der Blutbehandlungsvorrichtung umfasste Erfassungseinrichtung ist programmiert und/oder konfiguriert zur Durchführung des erfindungsgemäßen Verfahrens, insbesondere nach erfolgtem Bereitstellen der Blutbehandlungsvorrichtung und im Zusammenwirken mit der Blutbehandlungsvorrichtung.

Die erfindungsgemäße Blutbehandlungsvorrichtung weist wenigstens eine offenbarte Erfassungseinrichtung auf und/oder ist hiermit in Signalübertragung verbunden oder steht mit dieser in einer Signalübertragungsbeziehung.

Der offenbarte Blutschlauchsatz dient dazu, bei einer Blutbehandlung mittels einer Blutbehandlungsvorrichtung, und insbesondere in einem erfindungsgemäßen Verfahren, verwendet zu werden. Der offenbarte Blutschlauchsatz weist wenigstens eine Druckmessleitung, einen Verbinder zum Verbinden der Druckmessleitung mit der Blutbehandlungsvorrichtung und wenigstens eine in der Druckmessleitung oder im Verbinder angeordnete luftdurchlässige Membran auf. Eine luftdurchlässige Membran lässt einen Luftstrom bzw. Gasstrom durch die Membran hindurch zu.

Ein offenbartes digitales, insbesondere nicht-flüchtiges, Speichermedium, insbesondere in Form eines maschinenlesbaren Trägers, insbesondere in Form einer Diskette, CD, DVD oder EPROM, insbesondere mit elektronisch oder optisch auslesbaren Steuersignalen, kann derart mit einem programmierbaren Computersystem zusammenwirken, dass die maschinellen Schritte eines erfindungsgemäßen Verfahrens veranlasst werden.

Ein offenbartes Computerprogramm-Produkt weist einen volatilen, flüchtigen oder auf einem maschinenlesbaren Träger gespeicherten Programmcode oder eine Signalwelle zur Veranlassung der maschinellen Schritte des erfindungsgemäßen Verfahrens, wenn das Computerprogramm-Produkt auf einem Rechner abläuft, auf. Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein offenbartes Computerprogramm weist einen Programmcode auf zur Veranlassung der maschinellen Schritte des erfindungsgemäßen Verfahrens, wenn das Computerprogramm auf einem Computer abläuft. Unter einem Computerprogramm kann erfindungsgemäß beispielsweise ein physikalisches, vertriebsfähiges Software-Produkt verstanden werden, welches ein Programm aufweist.

Für das offenbarte digitale Speichermedium, das offenbarte Computerprogramm-Produkt und das offenbarte Computerprogramm gilt, dass alle, einige oder manche der maschinell durchgeführten Schritte des erfindungsgemäßen Verfahrens veranlasst werden. Dies gilt insbesondere im Zusammenwirken mit einer von der Blutbehandlungsvorrichtung umfassten Erfassungsvorrichtung und/oder einer bereitgestellten Blutbehandlungsvorrichtung wie hierin beschrieben.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

In einigen erfindungsgemäßen, beispielhaften Ausführungsformen ist das Auswerten ein Vergleichen mit einem bekannten Druckwert oder einem Anfangsdruckwert, oder ein Bilden einer Differenz (beispielsweise gegenüber einem bekannten oder zuvor gemessenen Ausgangsdruck oder einem Kontrollwert, etwa einem Druck von 0 mmHg). Dabei kann ein absoluter Anstieg ermittelt werden, oder ein relativer oder prozentualer.

In bestimmten erfindungsgemäßen, beispielhaften Ausführungsformen ist eine maximale Druckschwelle vorgegeben. Diese liegt über einer Druckschwelle, welcher der gemessene Druck zum Treffen der Aussage erreichen muss, dass eine Verbindung vorliegt. Durch Vorsehen der maximalen Druckschwelle ist es optional möglich, einen kompletten Verschluss der Druckluftleitung der Blutbehandlungsvorrichtung festzustellen. Ein solcher kompletter Verschluss könnte beispielsweise auf eine defekte geräteseitige luftdurchlässige Membran (z. B., siehe unten, oder z. B. Hydrophobfilter) zurückzuführen sein und vorteilhafterweise hierauf schließen lassen.

In manchen erfindungsgemäßen, beispielhaften Ausführungsformen ist oder umfasst das Auswerten eines Anstiegs kein Auswerten eines Druckabfalls, beispielsweise kein Auswerten eines Abfallens des gemessenen Drucks gegenüber einem Ausgangswert oder einem zwischenzeitlich gemessenen Druckwert.

In bestimmten erfindungsgemäßen, beispielhaften Ausführungsformen ist der Drucksensor Teil einer Rücklaufdruckmesseinheit, mittels welcher auch der im Blutschlauchsatz herrschende venöse Blutdruck gemessen werden kann.

In manchen erfindungsgemäßen, beispielhaften Ausführungsformen ist oder umfasst die luftdurchlässige Membran eine Transducer-Protector-Membran (kurz: TP).

In bestimmten erfindungsgemäßen, beispielhaften Ausführungsformen weist die Druckmessleitung einen Verbinder, vorzugsweise einen Luer-Verbinder, auf, welcher mit dem Druckluftausgang der Blutbehandlungsvorrichtung verbindbar ist.

In bestimmten erfindungsgemäßen, beispielhaften Ausführungsformen ist die luftdurchlässige Membran im Verbinder, sofern vorhanden, angeordnet.

Der mittels des Drucksensors gemessene und ausgewertete Druckwert kann insbesondere der maximale oder höchste Druckwert sein, der gemessen wird, insbesondere bis zu einem Abbruch des Verfahrens wegen Zeitüberschreitung (*time-out*) falls vorgesehen. Die hierzu vorbestimmte, maximale Zeit, die es nicht zu überschreiten gilt, kann z. B. bis 3, 5, 7, 10 s (Sekunden) betragen. Bevorzugt ist jedoch, ein *time-out* auf 1 bis 2 s zu beschränken, insbesondere auf maximal 1 s. Diese kurze Zeitspanne erlaubt auch ein mehrfaches Wiederholen des erfindungsgemäßen Verfahrens, insbesondere bevor der Blutschlauchsatz angeschlossenen wird und damit nicht mehr als steril gilt.

In manchen erfindungsgemäßen, beispielhaften Ausführungsformen ist oder umfasst das Auswerten des Anstiegs des gemessenen Drucks oder dessen Anstiegs über der Zeit ein Treffen einer Aussage über die Verbindung zwischen dem Druckluftausgang und der Druckmessleitung anhand der Auswertung des Anstiegs.

Diese Aussage kann optional quantitativ oder qualitativ sein. Sie kann beispielsweise die Ausprägungen "korrekt", "korrekt verbunden", "nicht verbunden" und/oder "verbunden, aber nicht korrekt" zum Ergebnis haben.

In manchen erfindungsgemäßen Ausführungsformen ist die Aussage über die Verbindung die Feststellung, dass eine Verbindung zwischen Druckluftausgang und Druckmessleitung besteht oder nicht besteht, und/oder dass sie fehlerhaft oder korrekt ist.

In einigen erfindungsgemäßen, beispielhaften Ausführungsformen kann eine "Verbindung" als Verbindungszustand verstanden werden. Sie kann eine mechanische Verbindung mittels zweier oder mehr Verbindungselemente sein.

In einigen erfindungsgemäßen, beispielhaften Ausführungsformen umfasst das Verfahren ein Bereitstellen einer abgeschlossenen Sammlung (etwa als Liste, als Datei, als Teil einer Datenbank, oder dergleichen) an Mindestwerten, Maximalwerten, Mustern, Kennlinien, Druck-Volumenstrom-Kennlinie, Druck-Anströmgeschwindigkeits-Kennlinie und/oder Verläufen von Drücken oder Druckanstiegen über der Zeit, welche für zwei oder mehr vorbestimmte, voneinander verschiedene Druckmessleitungen oder Bauarten, Typen oder Modelle von Druckmessleitungen erhoben wurden oder diese kennzeichnen, und welche vorzugsweise für die vorbestimmten Druckmessleitungen, insbesondere eineindeutig, kennzeichnend sind.

In diesen Ausführungsformen umfasst oder ist das Auswerten des Anstiegs des gemessenen Drucks oder dessen Verlaufs über der Zeit ein Überprüfen (oder besteht hieraus), ob der Anstieg oder der Verlauf (oder ein Muster, eine Kennlinie oder ein Verlauf hiervon) in der abgeschlossenen Sammlung enthalten ist. Ein optionales Ausgeben des Ergebnisses dieser Auswertung kann vorgesehen sein.

In einigen erfindungsgemäßen, beispielhaften Ausführungsformen erfolgt das Aufbauen des Drucks mittels der Drucklufteinrichtung wiederholt, z. B. in vorbestimmten Zeitabständen.

In einigen erfindungsgemäßen, beispielhaften Ausführungsformen erfolgt das Aufbauen des Drucks mittels der Drucklufteinrichtung fortlaufend, d. h. von Beginn des Verfahrens bis zu dessen Abbruch. Die Drucklufteinrichtung liefert in diesen Ausführungsformen somit durchgehend Überdruck und baut folglich Druck auf. Sie liefert einen stets positiven (d. h. größer Null) Volumenstrom.

Ein Abbruch des Verfahrens erfolgt beispielsweise, wenn das Verfahren zur Aussage geführt hat, dass eine korrekte Verbindung vorliegt, was daran erkannt wurde, dass der gemessene Druck bis auf oder über einen vorbestimmten Schwellenwert angestiegen ist.

Ein Abbruch des Verfahrens erfolgt beispielsweise, wenn das Verfahren es innerhalb einer vorbestimmten Zeitdauer nicht erlaubt die Aussage zu treffen, dass eine korrekte Verbindung vorliegt. Letzteres kann daran erkannt werden, dass der gemessene Druck bis Ablauf der vorbestimmten Zeitdauer nicht auf oder nicht über einen vorbestimmten Schwellenwert angestiegen ist.

In gewissen erfindungsgemäßen, beispielhaften Ausführungsformen ist der Schwellenwert, welcher vom gemessenen Druck zum Bestehen des erfindungsgemäßen Testverfahrens erreicht werden muss, in Kenntnis des verwendeten Blutschlauchsatzes oder der verwendeten Druckmessleitung vorbestimmt. Der Schritt des Vorbestimmens kann Teil des erfindungsgemäßen Verfahrens sein. Er kann weiter optional automatisiert sein, beispielsweise indem die Blutbehandlungsvorrichtung abfragt, welcher Art (Typ, Modell), der verwendete Blutschlauchsatz oder die verwendete Druckmessleitung ist.

In manchen erfindungsgemäßen, beispielhaften Ausführungsformen erfolgt kein Anlegen eines Unterdrucks am Druckluftausgang und/oder in der Druckmessleitung.

In einigen erfindungsgemäßen, beispielhaften Ausführungsformen ist oder ergibt das Treffen einer Aussage über die Verbindung, dass eine Verbindung nicht oder nicht korrekt besteht, falls ein vorbestimmter Mindestdruckanstieg, ein vorbestimmter Mindestdruck oder ein vorbestimmter Verlauf des Druckanstiegs über der Zeit nicht erreicht oder erkannt wird.

In bestimmten erfindungsgemäßen, beispielhaften Ausführungsformen erfolgt kein Druckhaltetest.

In einigen erfindungsgemäßen, beispielhaften Ausführungsformen hat der Blutschlauchsatz in der Druckmessleitung keinen Druckdom.

In gewissen erfindungsgemäßen, beispielhaften Ausführungsformen hat der Blutschlauchsatz in der Druckmessleitung kein luftundurchlässiges Diaphragma, welches beispielsweise von einem Druckdom bekannt ist.

In manchen erfindungsgemäßen, beispielhaften Ausführungsformen umgibt der Blutschlauchsatz in der Druckmessleitung kein geschlossenes Volumen. Vorzugsweise ist die Druckmessleitung in diesen Ausführungsformen vielmehr an einem Ende (dem nicht mit dem Druckluftausgang verbundenen Ende) hiervon offen oder mündet in ein ausreichend großes Volumen wie jenes des Blasenfängers oder der venösen Blutkammer. Man kann hier von einer "open-line" sprechen.

In einigen erfindungsgemäßen, beispielhaften Ausführungsformen ist die Druckmessleitung zu einer venösen oder arteriellen Patientenleitung oder anderen Leitung des Blutschlauchsatzes offen.

In manchen erfindungsgemäßen, beispielhaften Ausführungsformen ist oder ergibt das Treffen einer Aussage über die Verbindung, dass die Verbindung nicht oder nicht korrekt besteht, falls der gemessene Druckanstieg einen vorbestimmten Druckwert nicht erreicht oder nicht übersteigt oder unter dem vorbestimmten Druckwert bleibt.

In einigen erfindungsgemäßen, beispielhaften Ausführungsformen ist oder ergibt das Treffen einer Aussage über die Verbindung, dass die Verbindung nicht oder nicht korrekt besteht, falls der gemessene Druck nicht innerhalb einer vorbestimmten Zeitdauer (welche beispielsweise mit dem Aufbringen des Drucks und der Luftströmung durch die Drucklufteinrichtung beginnt) einen vorbestimmten Druckwert erreicht oder übersteigt (hierin auch als sog. *time-out* bezeichnet).

In einigen erfindungsgemäßen, beispielhaften Ausführungsformen wird bei Treffen einer Aussage, dass die Verbindung nicht oder nicht korrekt besteht, eine Fehlermeldung (beispielsweise optisch und/oder akustisch) ausgegeben und/oder das Verfahren wird abgebrochen.

In einigen erfindungsgemäßen, beispielhaften Ausführungsformen wird das erfindungsgemäße Verfahren einer beliebigen Ausführungsform wiederholt, falls das Treffen einer Aussage über die Verbindung ist oder ergibt, dass die Verbindung nicht oder nicht korrekt besteht, oder falls der Benutzer dies fordert (etwa durch Betätigen eines Betätigungselements wie eines Schalter, einer Touch-Screenfläche oder dergleichen).

In einigen erfindungsgemäßen, beispielhaften Ausführungsformen umfasst das Verfahren ein Unterbinden der Aufnahme einer Blutbehandlung mittels der bereitgestellten Blutbehandlungsvorrichtung. Dies kann insbesondere für eine solche Blutbehandlung gelten, bei welcher der extrakorporale Blutschlauchsatz bestimmungsgemäß verwendet wird oder verwendet werden soll. Das Unterbinden erfolgt in diesen Ausführungsformen, falls das Treffen einer Aussage über die Verbindung ergibt, dass die Verbindung nicht oder nicht korrekt besteht oder erfolgt ist, oder dass der Anstieg des Drucks oder dessen Verlauf nicht in der abgeschlossenen Sammlung enthalten ist.

In einigen erfindungsgemäßen, beispielhaften Ausführungsformen umfasst das Verfahren ein Sperren von manchen oder allen Behandlungsmodalitäten der bereitgestellten Blutbehandlungsvorrichtung und/oder Einschränken von Behandlungsparametern von mittels der bereitgestellten Blutbehandlungsvorrichtung durchführbaren Blutbehandlungsverfahren. Das Sperren erfolgt in diesen Ausführungsformen, falls das Treffen einer Aussage über die Verbindung ergibt, dass die Verbindung nicht oder nicht korrekt besteht oder erfolgt ist, oder dass der Anstieg des Drucks oder dessen Verlauf nicht in der abgeschlossenen Sammlung enthalten ist.

Erfindungsgemäß wird das Verfahren vor Aufnahme einer Behandlung eines Patienten, bei welcher der extrakorporale Blutschlauchsatz bestimmungsgemäß verwendet werden soll, beendet.

In einigen erfindungsgemäßen, beispielhaften Ausführungsformen sind die erfindungsgemäße oder die bereitgestellte Blutbehandlungsvorrichtung eine Vorrichtung zur Apherese oder Dialyse, wiederum insbesondere zur Hämodialyse, Hämofiltration oder Hämodiafiltration.

In einigen erfindungsgemäßen, beispielhaften Ausführungsformen weist die von der Blutbehandlungsvorrichtung umfasste Erfassungseinrichtung wenigstens eine Anzeigeeinrichtung zum Anzeigen eines Ergebnisses der Durchführung des Verfahrens auf oder ist hiermit in Signalübertragung verbunden. Die Anzeigeeinrichtung kann ein Display, eine Fehleranzeige oder dergleichen sein.

In einigen erfindungsgemäßen, beispielhaften Ausführungsformen weist die von der Blutbehandlungsvorrichtung umfasste Erfassungseinrichtung wenigstens eine Alarmeinrichtung auf, welche konfiguriert oder programmiert ist zum Ausgeben eines Alarms für den Fall, dass das Ergebnis der Durchführung des Verfahrens ist, dass die Verbindung nicht oder nicht korrekt besteht. Alternativ steht sie mit einer solchen Alarmeinrichtung in Signalübertragung.

In einigen erfindungsgemäßen, beispielhaften Ausführungsformen ist die von der Blutbehandlungsvorrichtung umfasste Erfassungseinrichtung programmiert und/oder konfiguriert, um derart auf die bereitgestellte Blutbehandlungsvorrichtung einzuwirken, dass wenigstens eine Behandlungsoption, zu welcher die Blutbehandlungsvorrichtung konstruktionsbedingt einsetzbar ist, vorübergehend nicht ausführbar ist, und/oder dass das Behandeln unter vorbestimmten Behandlungsparametern mittels der Blutbehandlungsvorrichtung vorübergehend nicht durchführbar ist, falls das Ergebnis der Durchführung des Verfahrens ist, dass die Verbindung nicht oder nicht korrekt besteht, oder dass der Anstieg oder der Verlauf in der abgeschlossenen Sammlung nicht enthalten ist.

In einigen erfindungsgemäßen, beispielhaften Ausführungsformen ist die von der Blutbehandlungsvorrichtung umfasste Erfassungseinrichtung eine Steuerungsvorrichtung und/oder ein Funktionstestmonitor oder weist dergleichen jeweils auf.

In einigen erfindungsgemäßen, beispielhaften Ausführungsformen weist die Blutbehandlungsvorrichtung eine luftdurchlässige Membran in der Druckluftleitung auf.

Ein Messen eines Drucks oder einer Druckveränderung kann jede Art von Feststellen umfassen, beispielsweise ein Messen mittels des Drucksensors, aber auch ein Errechnen oder ein Schließen ausgehend von Druckwerten, welche vom Drucksensor geliefert werden.

In manchen erfindungsgemäßen Ausführungsformen weisen die hierin beschriebenen Einrichtungen, Vorrichtungen, Apparate und andere Gegenstände die zum Ausführen von hierin beschriebenen Verfahrensschritten oder Testschritten erforderlichen, vorzugsweise nach dem jeweiligen Schritt benannten Einrichtungen, Vorrichtungen, Apparate oder anderen Gegenstände auf oder sind hiermit verbunden.

Die von der Blutbehandlungsvorrichtung umfasste Erfassungseinrichtung weist in bestimmten erfindungsgemäßen Ausführungsformen wenigstens eine Alarmeinrichtung zum Ausgeben eines Alarms auf. Die Alarmeinrichtung kann vorgesehen oder konfiguriert sein zum Ausgeben eines Alarms für den Fall, dass die Aussage getroffen wird, dass keine Verbindung oder keine korrekte Verbindung vorliegt. Der Alarm kann ein akustischer und/oder ein optischer Alarm sein.

Einige oder alle erfindungsgemäßen Ausführungsformen können einen oder mehrere der oben oder im Folgenden genannten Vorteile aufweisen.

So kann, da erfindungsgemäß sicherheitsrelevante Funktionen des verwendeten Blutschlauchsatzes, nämlich dessen Verbindung zum Zweck der Druckmessung, sowie der Blutbehandlungsvorrichtung geprüft werden, eine erhöhte Sicherheit für den Patienten geschaffen werden.

Da das erfindungsgemäße Verfahren beim Vorbereiten oder Aufrüsten der Blutbehandlungsvorrichtung erfolgen kann, kann ein technischer Fehler bereits erkannt werden, bevor der Patient konnektiert ist und bevor Blut in Kontakt mit der Blutbehandlungsvorrichtung sowie dem extrakorporalen Blutkreislauf oder Blutschlauchsatz gelangt ist. Letzteres vermeidet einen unnötigen Mehrverbrauch an Einwegartikeln. Es erlaubt ferner ein einfaches, da frühzeitiges Austauschen von defekten Druckmessleitungen oder Blutschlauchsätzen im Fehlerfall.

Da das erfindungsgemäße Verfahren in einigen erfindungsgemäßen Ausführungsformen ohne jedes Zutun des diensthabenden Personals erfolgen kann und automatisch abläuft, ist es vorteilhaft möglich, Fehler arbeitszeitsparend und bereits vor Behandlungsbeginn zu erkennen. Zudem erlaubt das hierin beschriebene Vorgehen dadurch, dass es automatisiert ausführbar ist, dass relevante Testschritte oder -prozeduren nicht vergessen werden können.

Die vorliegende Erfindung wird im Folgenden anhand der beigefügten Zeichnungen, in welchen identische Bezugszeichen gleiche oder ähnliche Bauteile bezeichnen, exemplarisch erläutert. In den zum Teil stark vereinfachten Figuren gilt:
- Fig. 1: zeigt ein schematisch vereinfachtes Schaubild einer erfindungsgemäßen Blutbehandlungsvorrichtung und einer Druckmessleitung eines offenbarten Blutschlauchsatzes;
- Fig. 2: zeigt ein Diagramm, in welchem der mittels des Drucksensors aus Fig. 1 erfindungsgemäß gemessene Druck über der Zeit eine korrekte Verbindung zwischen Druckmessleitung und Druckluftausgang zeigt;
- Fig. 3: zeigt in dem aus Fig. 2 bekannten Diagramm einen Druckverlauf bei nicht erfolgter Verbindung zwischen Druckmessleitung und Druckluftausgang; und
- Fig. 4: zeigt in dem aus Fig. 2 bekannten Diagramm einen weiteren Druckverlauf bei nicht korrekt erfolgter Verbindung zwischen Druckmessleitung und Druckluftausgang.

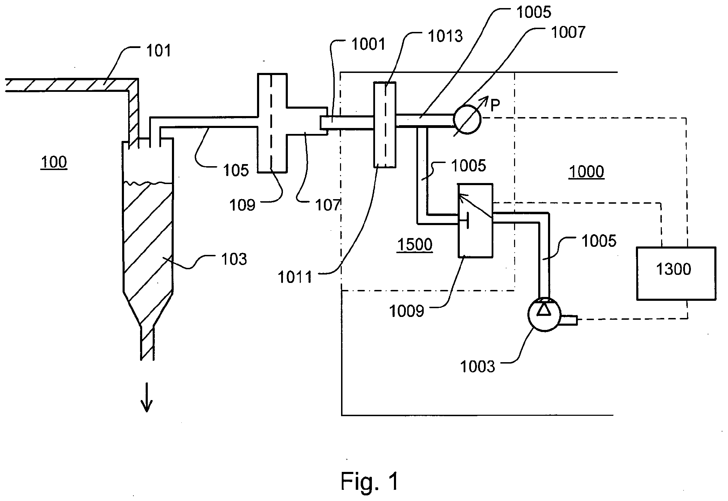

**Fig. 1** zeigt schematisch sehr vereinfacht Abschnitte einer erfindungsgemäßen Blutbehandlungsvorrichtung 1000 sowie Abschnitte eines offenbarten Blutschlauchsatzes 100.

Der extrakorporale Blutschlauchsatz 100, welcher optional außerhalb und innerhalb einer nicht dargestellten Blutkassette verlaufen kann, weist eine venöse Patientenleitung 101 und optional einen venösen Blasenfänger oder eine venöse Blutkammer 103 auf. Eine Durchströmung der Patientenleitung 101 kann bei deren Gebrauch in der Richtung des angegebenen Pfeils in Richtung Patient erfolgen.

Mit dem Blutschlauchsatz 100 verbunden (oder Teil hiervon) ist eine Druckmessleitung 105. Diese geht in Fig. 1 rein exemplarisch von der venösen Blutkammer 103 ab. Die Druckmessleitung 105 kann die Rücklaufdruckmessleitung sein. Sie kann eine "open line" sein.

Die Druckmessleitung 105 weist einen Verbinder 107 auf, welcher zum Verbinden der Druckmessleitung 105 mit einem Druckluftausgang 1001 der Blutbehandlungsvorrichtung 1000 vorgesehen und ausgestaltet ist. Rein optional sind der Verbinder 107 und der Druckluftausgang 1001 weibliche bzw. männliche Hälften eines Luer-Konnektors mit weiblichem bzw. männlichem Dichtkonus oder eines entsprechenden Luer-Lock-Konnektors mit zusätzlichem Sicherungsgewinde.

Der Druckluftausgang 1001 kann in oder an einer Außenwand der Blutbehandlungsvorrichtung 1000, beispielsweise in seiner Gehäusewand, liegen.

Der Verbinder 107 der Druckmessleitung 105, oder ein anderer Abschnitt der Druckmessleitung 105, welcher in einem im Gebrauchszustand der Druckmessleitung 105 von Luft durchströmten oder von Luft durchströmbaren Bereich der Druckmessleitung 105 liegt, weist eine luftdurchlässige Membran 109 auf. Die luftdurchlässige Membran 109 ist, rein beispielhaft, als hydrophobe luftdurchlässige Membran oder Hydrophobfilter ausgestaltet.

Die Blutbehandlungsvorrichtung 1000 weist einen Kompressor 1003 als Beispiel für eine Drucklufteinrichtung oder -quelle auf.

Kompressor 1003 und Druckluftausgang 1001 sind mittels einer Druckluftleitung 1005 in Fluidkommunikation verbunden. Der Kompressor 1003 kann optional weitere Ventile für einen anderen als seinen hierin beschrieben Einsatz aufweisen.

In oder an der Druckluftleitung 1005 sind ein Drucksensor 1007 und, rein optional, ein Umschaltventil 1009 (alternativ oder ergänzend eine Drossel, ein Schalter, eine Sperre und/oder dergleichen) vorgesehen.

Wie aus Fig. 1 erkannt werden kann, ist der Drucksensor 1007 derart in die Druckluftleitung 1005 eingebunden oder steht mit dieser in geeigneter Fluidkommunikation, dass er bei entsprechend geschaltetem Umschaltventil 1009 (falls vorhanden) und aktivem, d. h. eingeschaltetem Kompressor 1003 den aufgrund des Betriebs des Kompressors 1003 in der Druckluftleitung 1005 herrschenden Druck P messen kann. Der Drucksensor 1007 kann der Rücklaufdrucksensor sein.

Rein beispielhaft führt die Druckluftleitung 1005 durch einen optional vorgesehenen Schutzfilter 1011, welcher wiederum eine, vorzugsweise hydrophobe, im Strömungslauf liegende luftdurchlässige Membran 1013 aufweist. Ist eine solche luftdurchlässige Membran 1013 vorgesehen, so kann die Erfassungseinrichtung 1300 optional konfiguriert oder programmiert sein, um den Druckwiderstand, den die luftdurchlässige Membran 1013 darstellt, korrigierend bei Ablauf des erfindungsgemäßen Verfahrens zu berücksichtigen (etwa durch Filtern, Subtrahieren, usw.).

Die Blutbehandlungsvorrichtung 1000 weist eine Erfassungseinrichtung 1300 auf. Diese ist, wie mittels Strichlinien gezeigt, beispielhaft mit dem Kompressor 1003, dem Drucksensor 1007 und/oder dem Umschaltventil 1009 in Signalverbindung verbunden.

Wie mittels Strich-Punkt-Linien gezeigt, können wenigstens der Druckluftausgang 1001 und der Drucksensor 1007, ferner optional das Umschaltventil 1009 und der Schutzfilter 1011, soweit vorhanden, Teil der eigenständigen Druckmesseinheit 1500 sein, welche mit der Blutbehandlungsvorrichtung 1000 verbunden ist.

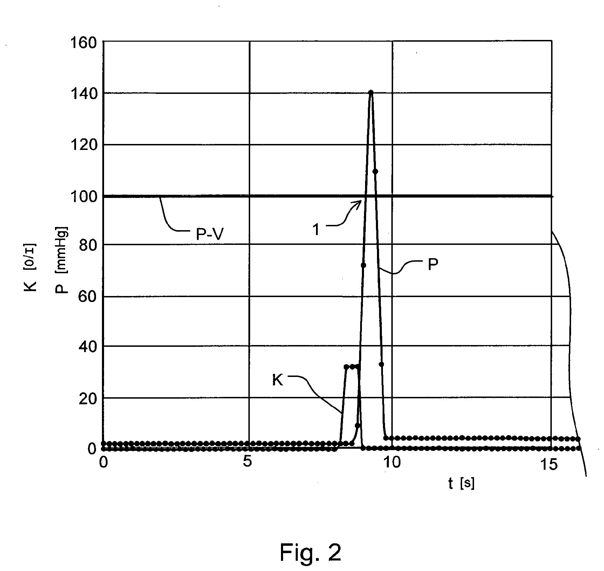

**Fig. 2** zeigt ein Diagramm, in welchem der Verlauf des mittels des Drucksensors 1007 aus Fig. 1 gemessenen Drucks P (hierin zur Abgrenzung als erster (Druck-)Verlauf verstanden), in der Einheit mmHg, über der Zeit t, in der Einheit s (Sekunden) aufgetragen ist.

Fig. 2 zeigt ferner einen zweiten Verlauf K, welcher den Zustand des Kompressors 1003 über der Zeit angibt. Dieser kann angeschaltet sein ("I") bzw. Druck und Luftstrom liefern, oder ausgeschaltet ("0") und keinen Druck und keinen Luftstrom liefern.

Aus Fig. 2 ist zu erkennen, dass der Kompressor 1003 etwa zum Zeitpunkt "8,4s" beginnt, Luft zu fördern. Als Reaktion hierauf steigt der Luftdruck in der Druckluftleitung 1005 (vergleiche Fig. 1) ab etwa dem Zeitpunkt "8,8s" an.

Wie Fig. 2 weiter zu entnehmen ist, steigt der Druck P rasant an und übersteigt kurz darauf einen Schwellenwert P-V (hier beispielhaft bei 100 mmHg). P-V ist im Beispiel der Fig. 2 das Maß für jenen Druck, ab welchem von einem Vorliegen einer korrekten Verbindung zwischen Druckmessleitung 105 und Druckluftausgang 1001 ausgegangen und somit die Aussage getroffen wird, dass eine korrekte Verbindung vorliegt. Der erfindungsgemäß durchgeführte Verbindungstest wird daher als erfolgreich angesehen.

Der Anstieg von P bis über P-V resultiert aus der Konfiguration des Strömungswiderstands des Verbinders 107 mit der luftdurchlässigen Membran 109 sowie der Druckmessleitung 105. Der Strömungswiderstand kann beispielsweise durch die Auswahl der Luftdurchlässigkeit der Membran 109 (Porengröße, Porenanteil, Dicke der luftdurchlässigen Membran), der freien Anströmfläche der luftdurchlässigen Membran 109, der inneren Geometrie der durchströmten Querschnitte des Verbinders 107 sowie dem Durchmesser und der Länge der Druckmessleitung 105 konfiguriert werden.

Fig. 2 zeigt ferner, dass bei der beispielhaften Ausführung des erfindungsgemäßen Verfahrens, welches in Fig. 2 gezeigt ist, der Kompressor 1003 bereits abgeschaltet wird, sobald der gemessene Druck P den Schwellenwert P-V erreicht hat. Der weitere Anstieg des Drucks P bis auf rund 140 mmHg ist im Beispiel der Fig. 2 nicht mehr relevant; das Verfahren endet zu dem Zeitpunkt, an dem der Druckanstieg auf 100 mmHg (gleich P-V) erkannt wurde.

Aufgrund des Abschaltens des Kompressors 1003 fällt der gemessene Druck P nach Beendigung des erfindungsgemäßen Verfahrens wieder auf seinen Ausgangswert von 0 mmHg ab. Das frühest mögliche Abschalten des Kompressors 1003, also z. B. sobald gemessen wird, dass P wenigstens den Wert P-V erreicht hat, kann dem Schutz der luftdurchlässigen Membran 109 dienen und daher von Vorteil sein.

Wie Fig. 2 auch zu entnehmen ist, findet erfindungsgemäß kein Druckhaltetest statt. Es findet vorzugsweise keine Auswertung des Drucks P hinsichtlich seines Abfallens statt. Es wird vorzugsweise kein Druckwert über die Zeit integriert. Es wird vorzugsweise nicht ein Nachlassen des mittels des Kompressors 1003 erzeugten Drucks ausgewertet.

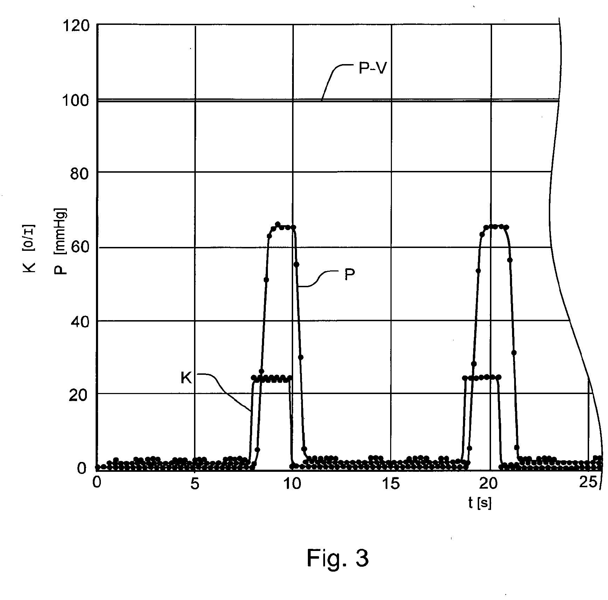

**Fig. 3** zeigt im Diagramm der Fig. 2 einen anderen Verlauf des mittels Drucksensor 1007 gemessenen Drucks P als jenen der Fig. 2.

Der in Fig. 3 gezeigte Verlauf des Drucks P erreicht in seinem Maximum von ca. 70 mmHg jedoch nicht den Schwellenwert P-V. Nachdem dies auch nach einer vorbestimmten Zeitdauer noch der Fall ist, wird das Verfahren abgebrochen und bei etwa "18s" wiederholt.

Der in Fig. 3 gezeigt Verlauf des Drucks P erreicht den Schwellenwert P-V deshalb nicht, weil der durch den Druck des Kompressors 1003 erzeugte Luftstrom bei nicht verbundener Druckmessleitung 105 auf einen vergleichsweise nur geringen Widerstand stößt. Zwar wirken der Druckluftausgang 1001 (z. B. durch dessen als Drossel wirkenden Verbinder oder Luer-Verbinder) und, falls vorhanden, auch die geräteseitige luftdurchlässige Membran 1013 als Widerstand bzw. bauen einen solchen auf. Dieser ist jedoch geringer als in Fig. 2, wo der Widerstand zusätzlich durch den Verbinder 107 und durch die luftdurchlässige Membran 109 bewirkt wird.

Der mittels des erfindungsgemäßen Verfahrens durchgeführte Test, ob eine korrekte Verbindung vorliegt, ist nicht bestanden: es liegt keine Verbindung vor.

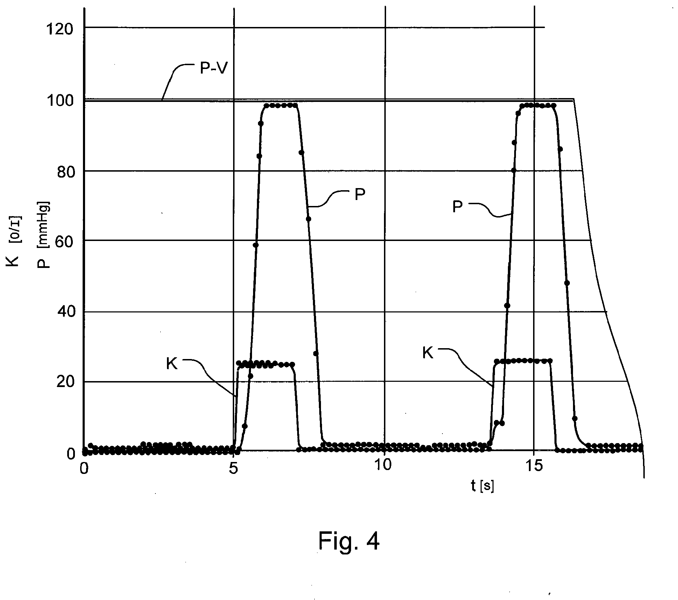

**Fig. 4** zeigt wiederum im Diagramm der Fig. 2 einen anderen mittels Drucksensor 1007 gemessenen Verlauf des Drucks P als jenen der Fig. 2 oder der Fig. 3.

Der in Fig. 4 gezeigte Druckverlauf P erreicht in seinem Maximum von ca. 95 mmHg wiederum nicht den Schwellenwert P-V. Nachdem dies auch nach einer vorbestimmten Zeitdauer noch der Fall ist, wird das Verfahren abgebrochen und bei etwa "14s" wiederholt.

Anders als bei Fig. 3 kann man dem Verlauf des Drucks P in Fig. 4 jedoch entnehmen, dass nicht nur der Widerstand des Verbinders 107 vorliegen kann, sondern, da in Fig. 4 ein höherer Wert P gemessen werden konnte als z. B. in Fig. 3, eine weitere Ursache für den gemessenen Druckanstieg vorliegen muss. Diese ergibt sich beim Verlauf der Fig. 4 aus dem Vorhandensein einer Verbindung zwischen Druckmessleitung 105 und Druckluftausgang 1001, welche jedoch - man vergleiche mit dem Verlauf der Fig. 2 - fehlerhaft sein muss und beispielsweise auf dem Vorliegen einer defekten luftdurchlässigen Membran 109, auf einer defekten Druckmessleitung 105 oder auf der Verwendung einer vom Hersteller der Blutbehandlungsvorrichtung nicht zur Druckmessung autorisierten Druckmessleitung 105 beruht.

Bei Bestehen und/oder bei Nichtbestehen von Verbindungstests können entsprechende Meldungen ausgegeben werden, wie vorstehend diskutiert.

In bestimmten erfindungsgemäßen Ausführungsformen sind der Verbinder 107 mit luftdurchlässiger Membran 109 und die Druckmessleitung 105 so konfiguriert, dass ein zusätzlicher Druckverlust in Höhe von 56 mmHg bis 93 mmHg auftritt bei Durchströmung der Elemente mit einem Normvolumenstrom mit Luft (Normbedingungen: 1 bar Umgebungsdruck; 293,15 K Umgebungstemperatur) zwischen 1,4 1/min und 2,1 1/min.

Die Länge der Druckmessleitung (vom Verbinder 107) bis zum Blasenfänger 103 beträgt zwischen 16,5 bis 27,5 cm; vorzugsweise 22 cm; der Außendurchmesser beträgt 5,5 mm; der Innendurchmesser 3,5 mm; der Werkstoff des Schlauchs ist vorzugsweise PVC (Polyvinylchlorid). Vorzugsweise ist eine dauerhafte Verklebung der Druckmessleitung in einem Stutzen am Verbinder 107 gewählt.

Vorzugsweise ist der Verbinder 107 des freien Endes die weibliche Hälfte eines Luer-Lock-Konnektors mit weiblichem Luer-Konus und umgebendem Außengewinde.

Vorzugsweise beträgt der Durchmesser des kleinsten freien Strömungsquerschnitts im weiblichen Luer-Konus 2,5 mm; der Werkstoff des Luer-Konus ist vorzugsweise PBT (Polybutylenterephthalat).

Die luftdurchlässige Membran 109 hat vorzugsweise einen freien Strömungsdurchmesser im Verbinder 107, der vorzugsweise senkrecht zur Durchströmungsrichtung steht, von 10 bis 14 mm, besonders bevorzugt 12 mm. Die Dicke der luftdurchlässigen Membran 109 beträgt vorzugsweise beispielsweise etwa 0,15 mm.

Der mittlere Porendurchmesser der luftdurchlässigen Membran 109 (deren Werkstoff unter anderen PTFE (Polytetrafluorethylen) sein kann, beträgt vorzugsweise zwischen 0,1 µm und 0,6 µm, besonders bevorzugt 0,2 µm (Mikrometer).

Die Membran ist im Verbinder, vorzugsweise beidseitig, optional von strahlenförmigen Stützrippen abgestützt, um die Luftdurchlässigkeit des gesamten freien Querschnitts zu gewährleisten.

Der Verbinder 107 weist vorzugsweise eine Farbmarkung auf (er ist beispielsweise zumindest teilweise aus blauem Kunststoff gefertigt) und der Druckluftausgang 1001 weist eine entsprechende Farbmarkierung (z. B. ein blaues Farbelement) auf, so dass dem Anwender zusätzlich ein optischer Hinweis zum Konnektieren gegeben wird. Dies vereinfacht das Aufrüsten des Blutschlauchsatzes 100 an der Blutbehandlungsvorrichtung 1000, verringert die Gefahr von Fehlern beim Aufrüsten und erhöht die Sicherheit des Patienten.

Die vorstehenden Zahlenangaben entsprechen jenen, bei welchen die Anmelderin besonders eindeutige Ergebnisse beim Durchführen des erfindungsgemäßen Verfahrens beobachten konnte. Sie sind daher vorteilhaft und bevorzugt.

Der maschinenseitige Druckluftausgang 1001 kann eine männliche Hälfte des Luer-Lock-Konnektors mit männlichem Luer-Konus und umgebendem Innengewinde sein. Er kann vorteilhaft, hygienisch einwandfrei da einfach zu reinigen, aus Edelstahl gefertigt sein.

Die Dauer einer erfolgreichen Messung kann in bestimmten erfindungsgemäßen Ausführungsformen und insbesondere bei der vorstehend beschriebenen Ausgestaltung von Verbinder 107 und luftdurchlässiger Membran 109 bei korrekter Konnektion des Verbinders 107 am Druckluftausgang 1001 1 bis 2 s betragen. Ein *time-out* kann beispielsweise auf weniger oder gleich 1 s festgelegt sein. Dies erlaubt eine zeitsparende Abfrage, ob die Verbindung korrekt ist.

### Bezugszeichenliste

- 100: Blutschlauchsatz

- 101: venöse Patientenleitung
- 103: Blasenfänger oder venöse Blutkammer
- 105: Druckmessleitung
- 107: Verbinder
- 109: luftdurchlässige Membran

- 1000: Blutbehandlungsvorrichtung

- 1001: Druckluftausgang
- 1003: Drucklufteinrichtung, z. B. Kompressor
- 1005: Druckluftleitung
- 1007: Drucksensor
- 1009: Umschaltventil
- 1011: Schutzfilter
- 1013: luftdurchlässige Membran

- 1300: Erfassungseinrichtung
- 1500: Druckmesseinheit

- P: gemessener Druck
- K: Einschaltzustand des Kompressors
- P-V: Schwellenwert für eine korrekte Verbindung
- t: Zeit

## Patentansprüche

1. Verfahren zum Überprüfen einer Verbindung zwischen einem Druckluftausgang (1001) einer Blutbehandlungsvorrichtung (1000) und einer Druckmessleitung (105) eines extrakorporalen Blutschlauchsatzes (100),
wobei das Verfahren die Schritte aufweist:
- Bereitstellen einer Blutbehandlungsvorrichtung (1000) mit
- einer Druckluftleitung (1005);
- einem mit der Druckluftleitung (1005) in Fluidkommunikation stehenden Kompressor (1003) zum Erzeugen von Druck und einer Luftströmung innerhalb der Druckluftleitung (1005);
- einem sowohl mit der Druckluftleitung (1005) als auch mit einem Äußeren der Blutbehandlungsvorrichtung (1000) in Fluidkommunikation stehenden Druckluftausgang (1001), wobei der Druckluftausgang (1001) ausgestaltet ist, um mit einer Druckmessleitung (105) verbindbar zu sein; und
- einem Drucksensor (1007), welcher zum Messen des am Druckluftausgang (1001) oder in der Druckluftleitung (1005) herrschenden Drucks (P) angeordnet ist;
- Aufbauen eines Drucks und einer Luftströmung in der Druckluftleitung (1005) und/oder am Druckluftausgang (1001), mittels des Kompressors (1003);
- Messen eines am Druckluftausgang (1001) oder in der Druckluftleitung (1005) herrschenden Drucks (P) oder einer Druckveränderung über der Zeit mittels des Drucksensors (1007); und
- Auswerten eines Anstiegs des gemessenen Drucks (P) oder eines Anstiegs des gemessenen Drucks über der Zeit, jeweils mittels eines Vergleichs des gemessenen Drucks (P) oder eines gemessenen Druckanstiegs mit einem Schwellenwert (P-V),
wobei der Kompressor (1003) bereits abgeschaltet wird, sobald der gemessene Druck (P) den Schwellenwert (P-V) erreicht hat,
wobei die Druckmessleitung an einem nicht mit dem Druckluftausgang verbundenen Ende offen ist oder in ein Volumen eines Blasenfängers (103) oder einer venösen Blutkammer (103) mündet,
wobei das Verfahren vor Aufnahme einer Behandlung eines Patienten, zu welcher der extrakorporale Blutschlauchsatz (100) bestimmungsgemäß verwendet werden soll, abgeschlossen wird.

2. Verfahren nach Anspruch 1, wobei das Auswerten des Anstiegs des gemessenen Drucks (P) oder dessen Anstiegs über der Zeit ein
- Treffen einer Aussage über die Verbindung zwischen dem Druckluftausgang (1001) und der Druckmessleitung (105) anhand der Auswertung des Anstiegs
ist oder umfasst, wobei bei Treffen einer Aussage, dass die Verbindung nicht oder nicht korrekt besteht, eine Fehlermeldung ergeht und/oder das Verfahren abgebrochen wird.

3. Verfahren nach Anspruch 2, wobei das Treffen einer Aussage über die Verbindung ist, oder ergibt, dass eine Verbindung nicht oder nicht korrekt besteht, falls ein vorbestimmter Mindestdruckanstieg bei der Auswertung nicht erreicht oder erkannt wird.

4. Verfahren nach einem der Ansprüche 2 oder 3, wobei das Treffen einer Aussage über die Verbindung ist, oder ergibt, dass die Verbindung nicht oder nicht korrekt besteht, falls der gemessene Druckanstieg einen vorbestimmten Druckwert (P-V) nicht erreicht oder nicht übersteigt.

5. Verfahren nach einem der Ansprüche 2 bis 4, wobei das Treffen einer Aussage über die Verbindung ist oder ergibt, dass die Verbindung nicht oder nicht korrekt besteht, falls der gemessene Druck (P) nicht innerhalb einer maximalen Zeitdauer seit Aufbringen des Drucks (P) einen vorbestimmten Druckwert (P-V) erreicht oder übersteigt.

6. Verfahren nach einem der vorangegangenen Ansprüche, mit dem Schritt:
- Erneutes Durchführen des Verfahrens gemäß einem der vorangegangenen Ansprüche, falls das Treffen einer Aussage über die Verbindung ist oder ergibt, dass die Verbindung nicht oder nicht korrekt besteht, oder falls der Benutzer dies fordert.

7. Verfahren nach einem der Ansprüche 2 bis 6, mit dem weiteren Schritt:
- Unterbinden der Aufnahme einer Blutbehandlung mittels der bereitgestellten Blutbehandlungsvorrichtung (1000);
oder mit dem weiteren Schritt:
- Sperren von Behandlungsmodalitäten der bereitgestellten Blutbehandlungsvorrichtung (1000) und/oder Einschränken von Behandlungsparametern von mittels der bereitgestellten Blutbehandlungsvorrichtung (1000) durchführbaren Blutbehandlungsverfahren;
jeweils falls das Treffen einer Aussage über die Verbindung ist oder ergibt, dass die Verbindung nicht oder nicht korrekt besteht.

8. Blutbehandlungsvorrichtung (1000) mit einer Erfassungseinrichtung (1300), programmiert und/oder konfiguriert zum Durchführen oder Veranlassen eines Verfahrens nach einem der Ansprüche 1 bis 7,
wobei die Blutbehandlungsvorrichtung (1000) aufweist:
- eine Druckluftleitung (1005);
- einen mit der Druckluftleitung (1005) in Fluidkommunikation stehenden Kompressor (1003) zum Erzeugen von Druck und einer Luftströmung innerhalb der Druckluftleitung (1005);
- einen sowohl mit der Druckluftleitung (1005) als auch mit einem Äußeren der Blutbehandlungsvorrichtung (1000) in Fluidkommunikation stehenden Druckluftausgang (1001), wobei der Druckluftausgang (1001) ausgestaltet ist, um mit einer Druckmessleitung (105) verbindbar zu sein; und
- einen Drucksensor (1007), welcher zum Messen des am Druckluftausgang (1001) oder in der Druckluftleitung (1005) herrschenden Drucks (P) angeordnet ist.

9. Blutbehandlungsvorrichtung (1000) nach Anspruch 8, aufweisend wenigstens eine Anzeigeeinrichtung zum Anzeigen eines Ergebnisses der Durchführung des Verfahrens nach einem der Ansprüche 1 bis 7.

10. Blutbehandlungsvorrichtung (1000) nach Anspruch 8 oder 9, aufweisend wenigstens eine Alarmeinrichtung, konfiguriert zum Ausgeben eines Alarms für den Fall, dass das Ergebnis der Durchführung des Verfahrens nach einem der Ansprüche 1 bis 7 ist oder ergibt, dass die Verbindung nicht oder nicht korrekt besteht.

11. Blutbehandlungsvorrichtung (1000) nach einem der Ansprüche 8 bis 10, welche programmiert und/oder konfiguriert ist, so dass wenigstens eine Behandlungsoption, zu welcher die Blutbehandlungsvorrichtung (1000) konstruktionsbedingt einsetzbar ist, nicht durchführbar ist, und/oder dass das Behandeln unter vorbestimmten Behandlungsparametern mittels der Blutbehandlungsvorrichtung (1000) nicht durchführbar ist, falls das Ergebnis der Durchführung des Verfahrens nach einem der Ansprüche 1 bis 7 ist oder ergibt, dass die Verbindung nicht oder nicht korrekt besteht.

12. Blutbehandlungsvorrichtung (1000) nach einem der Ansprüche 8 bis 11, welche eine Steuerungsvorrichtung und/oder einen Funktionstestmonitor aufweist.

## Claims

1. A method for checking a connection between a compressed air outlet (1001) of a blood treatment apparatus (1000) and a pressure measuring line (105) of an extracorporeal blood tubing set (100),
wherein the method comprises the steps:
- providing a blood treatment apparatus (1000) having:
- a compressed air line (1005);
- a compressor (1003) in fluid communication with the compressed air line (1005) for generating pressure and an air flow within the compressed air line (1005);
- a compressed air outlet (1001) in fluid communication with both the compressed air line (1005) and an exterior of the blood treatment apparatus (1000), wherein the compressed air outlet (1001) is designed to be connectable with a pressure measuring line (105); and
- a pressure sensor (1007) which is arranged to measure the pressure (P) prevailing at the compressed air outlet (1001) or in the compressed air line (1005);
- setting up a pressure and an air flow in the compressed air line (1005) and/or at the compressed air outlet (1001), by means of the compressor (1003);
- measuring a pressure (P) prevailing at the compressed air outlet (1001) or in the compressed air line (1005) or measuring a pressure change over time by means of the pressure sensor (1007); and
- evaluating an increase of the measured pressure (P) or an increase of the measured pressure over time, each time by means of comparing the measured pressure (P) or a measured pressure increase with a threshold value (P-V),
wherein the compressor (1003) is already switched off as soon as the measured pressure (P) has reached the threshold value (P-V),
wherein the pressure measuring line is open at one end not connected with the compressed air outlet or leads into a volume of a bubble trap (103) or a venous blood chamber (103),
wherein the method is completed before the start of a treatment of a patient for which the extracorporeal blood tubing set (100) is intended to be used.

2. The method according to claim 1, wherein the evaluation of the increase of the measured pressure (P) or its increase over time is or includes:
- making a statement about the connection between the compressed air outlet (1001) and the pressure measuring line (105) on the basis of the evaluation of the increase,
wherein an error message is issued and/or the method is aborted when a statement is made that the connection does not exist or is incorrect.

3. The method according to claim 2, wherein making a statement about the connection is, or results in that a connection does not exist or is incorrect, when a predetermined minimum pressure increase is not reached or detected during the evaluation.

4. The method according to anyone of claims 2 or 3, wherein making a statement about the connection is, or results in that the connection does not exist or is incorrect, when the measured pressure increase does not reach or does not exceed a predetermined pressure value (P-V).

5. The method according to anyone of claims 2 to 4, wherein making a statement about the connection is, or results in that the connection does not exist or is incorrect, when the measured pressure (P) does not reach or exceed a predetermined pressure value (P-V) within a maximum period of time since application of the pressure (P).

6. The method according to anyone of the preceding claims, with the step:
- executing again the method according to anyone of the preceding claims if the statement made about the connection is, or results in that the connection does not exist or is incorrect, or if the user so requests.

7. The method according to anyone of the claims 2 to 6, with the further step:
- preventing the start of a blood treatment by means of the provided blood treatment apparatus (1000);
or with the further step:
- blocking treatment modalities of the provided blood treatment apparatus (1000) and/or limiting treatment parameters of blood treatment methods that can be performed by means of the provided blood treatment apparatus (1000) ;
if the statement made about the connection is, or results in that the connection does not exist or is incorrect, respectively.

8. A blood treatment apparatus (1000) having a detection device (1300), programmed and/or configured to execute or initiate a method according to anyone of claims 1 to 7, wherein the blood treatment apparatus (1000) comprises:
- a compressed air line (1005);
- a compressor (1003) in fluid communication with the compressed air line (1005) for generating pressure and an air flow within the compressed air line (1005);
- a compressed air outlet (1001) in fluid communication with both the compressed air line (1005) and an exterior of the blood treatment apparatus (1000), wherein the compressed air outlet (1001) is designed to be connectable with a pressure measuring line (105); and
- a pressure sensor (1007) which is arranged to measure the pressure (P) prevailing at the compressed air outlet (1001) or in the compressed air line (1005).

9. The blood treatment apparatus (1000) according to claim 8, comprising at least one display device for displaying a result of the execution of the method according to anyone of claims 1 to 7.

10. The blood treatment apparatus (1000) according to claim 8 or 9, comprising at least one alarm device configured to issue an alarm in the event that the result of the execution of the method according to anyone of claims 1 to 7 is, or results in that the connection does not exist or is incorrect.

11. The blood treatment apparatus (1000) according to anyone of claims 8 to 10, which is programmed and/or configured so that at least one treatment option for which the blood treatment apparatus (1000) can be used according to its design, cannot be performed and/or so that the treatment under pre-determined treatment parameters cannot be performed by means of the blood treatment apparatus (1000), if the result of executing the method according to anyone of claims 1 to 7 is, or results in that the connection does not exist or is incorrect.

12. The blood treatment apparatus (1000) according to anyone of claims 8 to 11, which comprises a control device and/or a function test monitor.

## Revendications

1. Un procédé pour vérifier une connexion entre une sortie d'air comprimé (1001) d'un appareil de traitement du sang (1000) et un conduit de mesure de pression (105) d'un assortiment de tuyaux sanguins extracorporels (100),
où le procédé comprend les étapes suivantes:
- mettre à disposition un appareil de traitement du sang (1000) comprenant:
- un conduit d'air comprimé (1005);
- un compresseur (1003) en communication fluidique avec le conduit d'air comprimé (1005) pour générer une pression ainsi qu'un flux d'air à l'intérieur du conduit d'air comprimé (1005);
- une sortie d'air comprimé (1001) en communication fluidique à la fois avec le conduit d'air comprimé (1005) et avec l'extérieur de l'appareil de traitement du sang (1000), où la sortie d'air comprimé (1001) est conçue pour pouvoir être raccordée à un conduit de mesure de pression (105); et
- un capteur de pression (1007) agencé de façon à mesurer la pression (P) régnant à la sortie d'air comprimé (1001) ou dans le conduit d'air comprimé (1005);
- établir une pression et un flux d'air dans le conduit d'air comprimé (1005) et/ou à la sortie d'air comprimé (1001) au moyen du compresseur (1003);
- mesurer une pression (P) régnant à la sortie d'air comprimé (1001) ou dans le conduit d'air comprimé (1005), ou un changement de pression dans le temps au moyen du capteur de pression (1007); et
- évaluer une augmentation de la pression mesurée (P) ou une augmentation de la pression mesurée dans le temps, en comparant respectivement la pression mesurée (P) ou l'augmentation de la pression mesurée avec une valeur seuil (P-V),
où le compresseur (1003) est désactivé dès lors que la pression mesurée (P) a atteint la valeur seuil (P-V),
où le conduit de mesure de pression est ouvert à une extrémité non reliée à la sortie d'air comprimé ou débouche sur un volume d'un piège à bulles (103) ou d'une chambre à sang veineuse (103),
où le procédé est achevé avant le début du traitement d'un patient pour lequel l'assortiment de tuyaux sanguins extracorporels (100) est destiné à être utilisé.

2. Le procédé selon la première revendication, où l'évaluation de l'augmentation de la pression mesurée (P) ou
de son augmentation dans le temps consiste en, ou comprend:
- l'aboutissement à une conclusion sur la connexion entre la sortie d'air comprimé (1001) et le conduit de mesure de pression (105) sur la base de l'évaluation de l'augmentation,
où lorsqu'il est abouti à une conclusion selon laquelle la connexion n' existe pas ou est incorrecte, un message d'erreur est émis et/ou le procédé est interrompu.

3. Le procédé selon la revendication 2, où la conclusion sur la connexion est, ou indique, qu'une connexion n'existe pas ou est incorrecte, si une augmentation de pression minimale prédéterminée n'est pas atteinte ou détectée lors de l'évaluation.

4. Le procédé selon l'une quelconque des revendications 2 ou 3, où la conclusion sur la connexion est, ou indique, que la connexion n'existe pas ou est incorrecte, si l'augmentation de la pression mesurée n'atteint pas ou ne dépasse pas une valeur de pression prédéterminée (P-V).

5. Le procédé selon l'une quelconque des revendications 2 à 4, où la conclusion sur la connexion est, ou indique, que la connexion n'existe pas ou est incorrecte, si la pression mesurée (P) n'atteint pas ou ne dépasse pas une valeur de pression prédéterminée (P-V) dans une période de temps maximale depuis l'application de la pression (P).

6. Le procédé selon l'une quelconque des revendications précédentes, comprenant l'étape suivante:
- répéter l'exécution du procédé selon l'une quelconque des revendications précédentes si la conclusion concernant la connexion est, ou indique, que la connexion n'existe pas ou est incorrecte, ou si l'utilisateur le demande.

7. Le procédé selon l'une quelconque des revendications 2 à 6, comprenant l'étape supplémentaire suivante:
- empêcher le commencement d'un traitement du sang au moyen de l'appareil de traitement du sang mis à disposition (1000);
ou l'étape supplémentaire suivante:
- bloquer des modalités de traitement de l'appareil de traitement du sang mis à disposition (1000) et/ou limiter des paramètres de traitement du procédé de traitement du sang pouvant être effectués au moyen de l'appareil de traitement du sang mis à disposition (1000);
si la conclusion sur la connexion est, ou indique, que la connexion n'existe pas ou est incorrecte, respectivement.

8. Un appareil de traitement du sang (1000) ayant un dispositif de détection (1300), programmé et/ou configuré pour exécuter ou déclencher un procédé selon l'une quelconque des revendications 1 à 7, où le dispositif de traitement du sang (1000) comprend:
- un conduit d'air comprimé (1005);
- un compresseur (1003) en communication fluidique avec le conduit d'air comprimé (1005) pour générer une pression ainsi qu'un flux d'air à l'intérieur du conduit d'air comprimé (1005);
- une sortie d'air comprimé (1001) en communication fluidique à la fois avec le conduit d'air comprimé (1005) et avec l'extérieur de l'appareil de traitement du sang (1000), où la sortie d'air comprimé (1001) est conçue pour pouvoir être raccordée à un conduit de mesure de pression (105); et
- un capteur de pression (1007) agencé de façon à mesurer la pression (P) régnant à la sortie d'air comprimé (1001) ou dans le conduit d'air comprimé (1005).

9. L'appareil de traitement du sang (1000) selon la revendication 8, comprenant au moins un dispositif d'affichage pour afficher un résultat de l'exécution du procédé selon l'une quelconque des revendications 1 à 7.

10. L'appareil de traitement du sang (1000) selon la revendication 8 ou 9, comprenant au moins un dispositif d'alarme configuré de façon à émettre une alarme dans le cas où le résultat de l'exécution du procédé selon l'une quelconque des revendications 1 à 7 est, ou indique, que la connexion n'existe pas ou est incorrecte.

11. L'appareil de traitement du sang (1000) selon l'une quelconque des revendications 8 à 10, programmé et/ou configuré de telle sorte qu'au moins une option de traitement pour laquelle l'appareil de traitement du sang (1000) peut être utilisé en raison de sa conception, ne puisse être réalisée et/ou qu'un traitement selon des paramètres de traitement prédéterminés ne puisse être effectué au moyen de l'appareil de traitement du sang (1000), si le résultat de l'exécution du procédé selon l'une quelconque des revendications 1 à 7 est, ou indique, que la connexion n' existe pas ou est incorrecte.

12. L'appareil de traitement du sang (1000) selon l'une quelconque des revendications 8 à 11, qui comprend un dispositif de contrôle et/ou un moniteur de test de fonctionnement.
